# EUROPEAN PATENT APPLICATION

(11) **EP 3 943 089 A1**
(43) Date of publication of application: **26.01.2022**
(21) Application number: 19920436.3
(22) Date of filing: 22.10.2019
(51) Int. Cl.: A61K 31/704, A61K 31/715, A61K 35/618, A61K 35/65, A61K 36/185, A61K 36/233, A61K 36/258, A61K 36/344, A61K 36/346, A61K 36/424, A61K 36/481, A61K 36/484, A61K 36/69, A61K 36/734, A61K 38/02, A61K 38/44, A61K 47/36, A61K 47/42, A23L 2/39, A23L 33/105, A61K 8/63

(54) **FREEZE-DRIED FORMULATION, PREPARATION METHOD AND APPLICATION THEREOF**

(30) Priority: 21.03.2019 CN 201910217331
(71) Applicant: Li, Hewei, Beijing 101312 (CN)
(72) Inventor: Li, Hewei, Beijing 101312 (CN)
(74) Representative: Cabinet Beau de Loménie
(86) International application number: PCT/CN2019/112445
(87) International publication number: WO 2020/186747

(57) **Abstract**

A freeze-dried formulation and a preparation method and an application thereof are provided. The triterpenoid saponin has a mass percentage of 0.004-95% in the freeze-dried formulation, a binding agent has a mass percentage of 0.01-99% in the freeze-dried formulation; the freeze-dried formulation is freeze-dried by a primary prepared solution containing the triterpenoid saponin and the binding agent; when the triterpenoid saponin has a mass percentage of 0.001%-50% in the primary prepared solution, the binding agent has a mass percentage of 0.01%-50% in the primary prepared solution, and the mass of triterpenoid saponin increases with the increase of the mass of the binding agent; and when the triterpenoid saponin has a mass percentage of 50%-95% in the primary prepared solution, the binding agent has a mass percentage of 0.01%-20% in the primary prepared solution, and the mass of the triterpenoid saponin decreases with the increase of the mass of the binding agent. The freeze-dried formulation has the advantage of rapid disintegration when it is used as an oral preparation, a solid beverage or a non-washing skin care product.

## Description

### TECHNICAL FIELD

The present invention relates to the technical field of formulation, and in particular to a freeze-dried formulation, a preparation method an application thereof.

### BACKGROUND OF THE INVENTION

Freeze-dried formulation is a kind of formulation which is produced by the following steps: medicinal components (raw materials) and auxiliary components (adjuvants) are dissolved by a solvent (e.g., water) and prepared into a certain concentration of pre-stocking solution, and the pre-stocking solution is frozen at low temperature in a sterile closed environment, and the solvent (e.g., water) in a product is sublimated by reducing ambient pressure and slowly rising the product temperature to retain solid loose block-shaped or powdery drugs.

Triterpenoid saponin is a kind of chemical component in natural drugs, and is featured by complex structure, low content and strong pharmacological activity. Most of these components are soluble in water very readily, thermosensitive and the like. Relative to steroid saponin, due to the feature of thermal instability, acidic saponin in triterpenoid saponin basically exists in fresh materials only; the machining process of a product will promote the transformation of acidic saponins into steroid saponins. Therefore, acidic saponin in triterpenoid saponin is hardly kept and thus rarely applied in practical products.

Clinically, triterpenoid saponin has lots of pharmacological activities, such as, blood sugar reduction and lipid lowering. At present, most of the triterpenoid saponins are identified to obtain the structure information, and thus may be synthesized artificially; but the artificially synthesizing way will greatly improve the cost; meanwhile, the residues of solvent and intermediate products during the synthetic route, impurity control will become a potential risk.

Triterpenoid saponin is conventionally applied in the treatment of cardiovascular diseases, and clinically applied to multiple dosage forms, such as, injections, oral tablets and capsules. Due to thermal instability, acidic saponin in triterpenoid saponin is limited to a large extent in processing technology and storage. Due to the feature of foaming ability, most of the saponin-containing freeze-dried formulations have the defects of very limited (generally 1-50 mg/granule and specification is 0.4-1 ml/granule) drug loading capacity, poor disintegration and solubility (higher than 10 s, and even to 1-3 min), and slow dissolution (15-60 min).

### SUMMARY OF THE INVENTION

Directed to the problems, such as, small drug loading capacity, poor disintegration and solubility, and slow dissolution of the freeze-dried formulation existing in the prior art, the objective of the present invention provides a freeze-dried formulation; the freeze-dried formulation includes a triterpenoid saponin and a binding agent, or further includes an adjuvant. When the freeze-dried formulation is used as an oral formulation, the disintegration time is less than 3 s, and the dissolving-out amount within 1 min is greater than 90%; when the freeze-dried formulation is used as a solid beverage, the disintegration time is less than 10 s, the dissolution time is less than 15 s, and the dissolving-out amount within 1 min is greater than 90%.

Another aspect of the invention provides a preparation method of a freeze-dried formulation, and the freeze-dried formulation is prepared by means of direct freeze-drying or soft ice freeze-drying; the preparation method has simple process and is convenient for commercial popularization and application.

Another aspect of the invention provides an application of the freeze-dried formulation, and the freeze-dried formulation may be applied in the fields of daily chemicals, drugs, health care products and food.

Another aspect of the invention provides an extraction method of the triterpenoid saponin; the method will not break the pharmacological activity of the triterpenoid saponin and has the advantage of high extraction quantity; 0.24-0.54 g acidic saponin may be extracted from every 1 g saponin-containing crude drug; meanwhile, the extraction method has production feasibility, and a freeze-dried formulation with the corresponding function may be further processed.

To achieve the objective of the present invention, the technical solution adopted herein is as follows:
a freeze-dried formulation is provided, where a triterpenoid saponin has a mass percentage of 0.004-95% in the freeze-dried formulation, and a binding agent has a mass percentage of 0.01-99% in the freeze-dried formulation;
the freeze-dried formulation is freeze-dried by a primary prepared solution containing the triterpenoid saponin and the binding agent:
   when the triterpenoid saponin has a mass percentage of 0.001%-50% in the primary prepared solution, the binding agent has a mass percentage of 0.01%-50% in the primary prepared solution, and a mass of the triterpenoid saponin increases with an increase of a mass of the binding agent;
   when the triterpenoid saponin has a mass percentage of 50%-95% in the primary prepared solution, the binding agent has a mass percentage of 0.01%-20% in the primary prepared solution, and the mass of the triterpenoid saponin decreases with the increase of the mass of the binding agent.

A freeze-dried formulation is provided, the freeze-dried formulation is freeze-dried by a primary prepared solution containing the triterpenoid saponin and a binding agent, and the freeze-dried formulation is an oral formulation, a solid beverage or a non-washing skin care product.

When the freeze-dried formulation is the oral formulation, the triterpenoid saponin has a mass percentage of 0.004%-50% in the primary prepared solution, the binding agent has a mass percentage of 0.01-50% in the primary prepared solution; preferably, the triterpenoid saponin has a mass percentage of 0.004%-30% in the primary prepared solution, the binding agent has a mass percentage of 0.01%-35% in the primary prepared solution ; more preferably, the triterpenoid saponin has a mass percentage of 0.004%-20% in the primary prepared solution, and the binding agent has a mass percentage of 0.01%-30% in the primary prepared solution. More preferably, the triterpenoid saponin has a mass percentage of 10%-20% in the primary prepared solution, and the binding agent has a mass percentage of 1%-10% in the primary prepared solution.

When the freeze-dried formulation is a solid beverage (including granules, fast dissolving tablets, and the like), the triterpenoid saponin has a mass percentage of 50%-95% in the primary prepared solution, and the binding agent has a mass percentage of 0.01%-20% in the primary prepared solution. Preferably, the triterpenoid saponin has a mass percentage of 50%-85% in the primary prepared solution, the binding agent has a mass percentage of 0.01%-20% in the primary prepared solution; more preferably, the triterpenoid saponin has a mass percentage of 50%-80% in the primary prepared solution, and the binding agent has a mass percentage of 0.01%-20% in the primary prepared solution. More preferably, the triterpenoid saponin has a mass percentage of 70%-80% in the primary prepared solution, and the binding agent has a mass percentage of 0.01%-10% in the primary prepared solution.

When the freeze-dried formulation is a non-washing skin care product (including an essence, a cream or a body lotion), the triterpenoid saponin has a mass percentage of 0.004-30% in the primary prepared solution, and the binding agent has a mass percentage of 0.01-50% in the primary prepared solution. Preferably, the triterpenoid saponin has a mass percentage of 0.004-10% in the primary prepared solution, the binding agent has a mass percentage of 0.01-10% in the primary prepared solution; more preferably, the triterpenoid saponin has a mass percentage of 0.004-0.1% in the primary prepared solution, and the binding agent has a mass percentage of 0.01-3% in the primary prepared solution.

In the above technical solution, the oral formulation has a disintegration time less than 3 s, and a dissolving-out amount greater than 90% within 1 min; and the solid beverage has a disintegration time less than 10 s, a dissolution time less than 15 s, and a dissolving-out amount greater than 90% within 1 min.

When the freeze-dried formulation serves as an oral formulation, the freeze-dried formulation may be disintegrated once it is taken (disintegrated within 3 s with 0.1-0.3 ml saliva) and dissolved out rapidly (90% above within 1 min); when the freeze-dried formulation serves as a solid beverage (including granules, fast dissolving tablets, and the like), the freeze-dried formulation may be still disintegrated rapidly (dispersed within 10 s) when it is put to water (water temperature may be 20°C-50°C available), and rapidly dissolved evenly (granules have been not seen within 15 s), and dissolved out rapidly (90% above within 1 min).

In the above technical solution, the freeze-dried formulation further comprises an adjuvant, and the adjuvant has a mass percentage of 0.01-50% in the primary prepared solution, and the adjuvant is one or a combination of a framework propping agent, a disintegrating agent, a skin feeling improver, an antioxidant, a corrigent, an essence, a transmucosal/skin penetration enhancer or a pH regulator.

The framework propping agent is sugar (e.g., maltose, trehalose and microcrystalline cellulose), a sugar alcohol (e.g., mannitol, sorbitol, and xylitol), a C2-C12 amino acid (e.g., glycine, alanine, and glutamic acid), and an inorganic salt (e.g., sodium phosphate, and aluminium silicate), dextrin (maltodextrin), or a combination thereof; the disintegrating agent is selected from one of effervescing agent, anhydrous lactose, starch and amylase, celluloses and cellulase, gum and hemicellulase, gelatin and protease, alginates, carrageenase, sucrose and invertase or a mixture of several of them; the skin feeling improver is one of polymethylsilsesquioxane, cassava starch, modified cassava starch, corn starch, modified corn starch, pearl powder, silica, titanium dioxide, mica, polydimethylsiloxane, di-C12-13 alkyl malate, dimyristyl tartrate, PPG-15 stearyl ether, chinlon-12, Vaccinium myrtillus seed oil, and other skin feeling improving matters or a mixture of several of them; the antioxidant is one of vitamin C, vitamin E, 2,6-di-t-butyl-4-methylphenol, tert-butylhydroquinone, butyl hydroxyanisole, N-phenylacetyl-L-glutamine, trihydroxybutyrophenone, citric acid, phosphoric acid derivative, EDTA, baicalin, astaxanthin, sodium sulfite, sodium pyrosulfite, gallate, phytic acid, tea polyphenol, anthocyanin, resveratrol, glutathione, superoxide dismutase, yeast/rice ferment filtrate, plant extract, beauveria bassiana extract, white truffle extract, fruit/vegetable extract, polyphenol compounds from plants and other antioxidative substances or a mixture of several of them; the corrigent and essence are selected from one of mint, chocolate flavor, fruit/vegetable flavor, flower/plant fragrance, plant flavor, vanilla flavor, coffee flavor, tea flavor, corn flavor, lemon flavor, milk flavor, fruity flavor, cranberry flavor, fructus crataegi flavor, Lycium barbarum flavor, and other essences or a mixture of several of them; the transmucosal/skin penetration enhancer is selected from one of lecithin, Tween, Span, or a mixture of several of them; the transdermal absorbent is selected from azone, lecithin, and ethoxy diethylene glycol or a mixture of several of them; and the pH regulator is selected from one of citric acid, sorbic acid, tartaric acid, lactic acid, malic acid, sodium bicarbonate, sodium carbonate, disodium hydrogen phosphate, calcium phosphate, potassium phosphate and trimagmesium phosphate or a mixture of several of them.

In the above technical solution, the binding agent consists of a freeze-dried binding agent and/or a low-temperature binding agent; the freeze-dried binding agent is selected from one or a combination of an artificial or a natural high-molecular polymer, a modified artificial or natural high-molecular polymer, an inorganic matter gel, a polysaccharide, a polysaccharide derivative and salt thereof, a sugar alcohol, a cellulose ether, modified starch, albumin or a polyamino acid; the low-temperature binding agent is one of a C1-C16 alcohol, a grease, a surfactant, an artificial or a natural high-molecular polymer, or a modified artificial or natural high-molecular polymer.

The artificial or natural high-molecular polymer is selected from collagen, gelatin, hydrolyzed gelatin, hydrolyzed collagen, arabic gum, xanthan gum, soyabean protein gum, Sclerotium gum, bio-saccharide gum, carrageenan, guar gum, gellan gum, pectin, konjac glucomannan, carrageenin, locust bean gum, gum, alginic acid, sodium alginate, agar, polyvinyl alcohol methyl acrylate grafted copolymer, Carbomer, Carbomer resin, polyvinylpyrrolidone, polyvinyl alcohol and a derivative thereof, polyethylene glycol and a derivative thereof, polyoxyethylene, polyacrylamide, sodium polyacrylate, polyacrylate, polyacrylic acid and a derivative thereof, or a combination thereof; the modified artificial or natural high-molecular polymer may be modified arabic gum, modified xanthan gum, modified guar gum, modified pectin, modified sodium polyacrylate and grafted starch, modified paraffin, or a combination thereof.

In the above technical solution, the freeze-dried formulation further comprises an active ingredient, and the active ingredient has a mass percentage of 0.01-50% in the primary prepared solution, and the active ingredient is one of steroid saponin, Astragalus extract, ginseng extract, American ginseng extract, Gynostemma pentaphyllum extract, flavone, ginseng polysaccharide, sea cucumber polysaccharide, amino acid, dencichine, pilose antler polypeptide, resveratrol, pearl powder, hydrolyzed pearl, rana japonica oil extract, SOD or hawthorn extract or a combination thereof.

In the above technical solution, the triterpenoid saponin is an acidic thermosensitive saponin, preferably, one or a combination of malonyl ginsenoside, Astragalus lanuginosus saponin, platycodin E, Codonopsis pilosula saponin A-G, Aster tataricus saponinHb, Condonpsis lanceolata saponin I-III or Gynostemma pentaphyllum saponin. The acidic thermosensitive saponin refers to triterpenoid saponin having a structure with carboxyl and a loss rate greater than 30% when the heating temperature is higher than 50°C; for the acidic thermosensitive saponin with high sensitivity, the loss rate thereof may be up to 30% after heating for 1-10 min; and for the acidic thermosensitive saponin with low sensitivity, the loss rate thereof may be up to 30% after heating for 1-48 h.

In the above technical solution, the triterpenoid saponin is purified and obtained from a saponin-containing crude drug by means of a CO₂ supercritical extraction process and a macroporous resin exchange adsorption process (the triterpenoid saponin has an extraction ratio of 80-90%), including the following steps:
1) taking, cleaning and cutting up the saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 min at -30°C-40°C by the CO₂ supercritical extraction process to obtain an extract C, and discarding residues, where an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 1;
5) taking the extract C and passing the extract C through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 2; and
6) separately using the triterpenoid saponin 1 and the triterpenoid saponin 2 or combining the triterpenoid saponins 1 and 2 to obtain a final triterpenoid saponin product.

In the above technical solution, the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, or Phytolacca acinosa or a combination thereof; the saponin crude drug is one or a combination of root, stem, leaf or flower of a plant. Preferably, the saponin-containing crude drug is the root of American ginseng, root of Panax notoginseng, root of ginseng, flower, leaf and stem of Panax notoginseng.

In the above technical solution, the freeze-dried formulation is prepared by the following method:
a) mixing the solvent (water is selected), the triterpenoid saponin and the binding agent to form a primary prepared solution in a form of a solution, an emulsion or a suspension; or mixing a solvent, the triterpenoid saponin, the binding agent and an adjuvant to form a primary prepared solution in a form of a solution, an emulsion or a suspension, then fixing a volume and degassing;
b) using a quantitative filling pump to pump the primary prepared solution obtained in the step a) into a quantitative forming die for degassing;
c) freeze-drying a side product obtained in the step b), and removing the solvent to obtain the freeze-dried formulation (a freeze-dried excipient);
or the freeze-dried formulation is prepared by the following steps:
a) preparation of a soft ice mixture:
   mixing the triterpenoid saponin, the binding agent and a solvent (water) to obtain a primary prepared solution, or mixing the triterpenoid saponin, the binding agent, a solvent and an adjuvant to form a primary prepared solution, then freezing the primary prepared solution to obtain a soft ice mixture 1;
b) mixing an active ingredient, the binding agent and a solvent (water) to obtain a primary prepared solution; or mixing an active ingredient, the binding agent, an adjuvant and a solvent (water) to obtain a primary prepared solution, then performing low-temperature cryogenic grinding or low-temperature spraying to obtain an ice powder 2;
c) using the active ingredient and the adjuvant as a dry powder 3;
d) using the active ingredient as a dry powder 4;
e) mixing one or more of the soft ice mixture 1, the ice powder 2, the dry powder 3 or the dry powder 4 to obtain a mixture of all soft ice;
f) performing shaping with a certain die to obtain a shaped mixture 5, and performing demolding;
g) freeze-drying the mixture 5 to obtain the freeze-dried formulation (a freeze-dried excipient).
or the freeze-dried formulation is prepared by the following steps:
a) mixing a solvent (water is selected), the triterpenoid saponin and the binding agent to form a primary prepared solution in a form of a solution, an emulsion or a suspension; or mixing a solvent, the triterpenoid saponin, the binding agent and an adjuvant to form a primary prepared solution in a form of a solution, an emulsion or a suspension, then fixing a volume and degassing;
b) using a quantitative filling pump, performing instillation in a capsule, wherein an internal temperature of the capsule is below an eutectic point of the solution, then rapidly freezing the solution when the solution drops to obtain a frozen solution;
c) freeze-drying the frozen solution to obtain the freeze-dried excipient.

In the above technical solution, the freeze-dried formulation may be in a shape of, but not limited to, a tablet shape, a spherical shape, an ellipsoidal shape, or various characters, animals, plants, food, graphic identifiers or cartoon images.

Another aspect of the invention further includes an application of the freeze-dried formulation, and the freeze-dried formulation may be applied in the fields of daily chemicals, drugs, health care products and food.

Another aspect of the invention further includes a barrier package, where the freeze-dried formulation is a content of the barrier package.

In the above technical solution, the barrier package is a double-aluminum, an aluminum plastic or a high-barrier high molecular blister package, a glass or metal container equipped with an aluminum plastic seal or a film seal, or a sealed container made of a metal. The aluminum plastic material is a composite material of PVDC, Aclar, EVOH and AL, or a composite material of aluminum and PP, PE, PET and PVC; the high-barrier high molecular material is PVDC, Aclar, EVOH; and the sealed container may be a box, tank, and bottle, for example, a pop can, an aluminium pot, a stainless steel tank. Or, the sealed container may be also a capsule coffee package or a small aluminum bowl, or the like.

Another aspect of the invention further includes an application of the triterpenoid saponin in an freeze-dried formulation; the triterpenoid saponin is an acidic thermosensitive saponin, and the acidic thermosensitive saponin is a triterpenoid saponin having a structure with carboxyl, a heating temperature higher than 50°C and a loss rate greater than 30%. For the acidic thermosensitive saponin with high sensitivity, the loss rate thereof may be up to 30% after heating for 1-10 min; and for the acidic thermosensitive saponin with low sensitivity, the loss rate thereof may be up to 30% after heating for 1-48 h.

Another aspect of the invention further includes an application of the triterpenoid saponin in a freeze-dried formulation; the triterpenoid saponin is purified and obtained from a saponin-containing crude drug by means of a CO₂ supercritical extraction process and a macroporous resin exchange adsorption process, including the following steps:
1) taking, cleaning and cutting up a saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 min at -30°C-40°C by the CO₂ supercritical extraction process to obtain an extract C, and discarding residues, where an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 1;
5) taking the extract C and passing the extract C through a macroporous resin column, washing with water to remove impurities, and eluting triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 2; and
6) separately using the triterpenoid saponin 1 and the triterpenoid saponin 2 or combining the triterpenoid saponins 1 and 2 to obtain a final triterpenoid saponin product.

In the above technical solution, the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, Astragalus membranaceus or Phytolacca acinosa; the saponin crude drug is one or a combination of root, stem, leaf or flower of a plant.

Another aspect of the invention further provides a method for extracting the triterpenoid saponin, characterized by including the following steps:
1) taking, cleaning and cutting up a saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 min at -30°C-40°C by the CO₂ supercritical extraction process to obtain an extract C, and discarding residues, where an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 1;
5) taking the extract C and passing the extract C through a macroporous resin column, washing with water to remove impurities, and eluting the triterpenoid saponin with 30%-100% ethyl alcohol, sampling and determining a target product by thin-layer chromatography, collecting the target segment, performing concentration under reduced pressure at 0°C-55°C until there is no alcoholic taste, and performing freeze-drying to obtain a triterpenoid saponin 2; and
6) separately using the triterpenoid saponin 1 and the triterpenoid saponin 2 or combining the triterpenoid saponins 1 and 2 to obtain a final triterpenoid saponin product.

The content of triterpenoid saponin in a saponin-containing crude drug is generally 30-60%; through the extraction method, 80-90% triterpenoid saponin may be extracted from the saponin-containing crude drug, thus achieving a high extraction ratio.

In the above technical solution, the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, Astragalus membranaceus or Phytolacca acinosa; the saponin crude drug is one or a combination of root, stem, leaf or flower of a plant.

Another aspect of the invention further includes a preparation method of the triterpenoid saponin, including the following steps:
a) mixing a solvent, a triterpenoid saponin and a binding agent to form a primary prepared solution in a form of a solution, an emulsion or a suspension; or mixing a solvent, a triterpenoid saponin, the binding agent and the adjuvant to form a primary prepared solution in the form of a solution, an emulsion or a suspension, then fixing the volume and degassing;
b) using a quantitative filling pump to pump the primary prepared solution obtained in the step a) into a quantitative forming die for degassing;
c) freeze-drying a side product obtained in the step b), and removing the solvent to obtain the freeze-dried formulation;
or the freeze-dried formulation is prepared by the following steps:
a) preparation of a soft ice mixture: mixing the triterpenoid saponin, the binding agent and the solvent to obtain a primary prepared solution, or mixing the triterpenoid saponin, the binding agent, the solvent and the adjuvant to form a primary prepared solution, then freezing the primary prepared solution to obtain a soft ice mixture 1;
b) mixing an active ingredient, the binding agent and the solvent to obtain a primary prepared solution; or mixing the active ingredient, the binding agent, the adjuvant and the solvent to obtain a primary prepared solution, then performing low-temperature cryogenic grinding or low-temperature spraying to obtain an ice powder 2;
c) using the active ingredient and the adjuvant as a dry powder 3;
d) using the active ingredient as a dry powder 4;
e) mixing one or more of the soft ice mixture 1, the ice powder 2, the dry powder 3 or the dry powder 4 to obtain a mixture of all soft ice;
f) performing shaping with a certain die to obtain a shaped mixture 5, and performing demolding;
g) freeze-drying the mixture 5 to obtain the freeze-dried formulation;
or the freeze-dried formulation is prepared by the following steps:
a) mixing the solvent, the triterpenoid saponin and the binding agent to form a primary prepared solution in the form of a solution, an emulsion or a suspension; or mixing the solvent, the triterpenoid saponin, the binding agent and an adjuvant to form a primary prepared solution in the form of a solution, an emulsion or a suspension, then fixing a volume and degassing;
b) using a quantitative filling pump, performing instillation in a capsule, where an internal temperature of the capsule is below an eutectic point of the solution, then rapidly freezing when the solution drops to obtain a frozen solution;
c) freeze-drying the frozen solution to obtain the freeze-dried formulation;

Compared with the prior art, the present invention has the following beneficial effects:
1. The addition of the triterpenoid saponin with a specific ratio renders the freeze-dried formulation of the present invention, as an oral formation, to have the advantage of rapid disintegration rate, and the disintegration time is less than 3 s. As a solid beverage, the freeze-dried formulation of the present invention has the advantages of fast disintegration rate, short disintegration time and rapid dissolution rate; the disintegration time is less than 3 s, the dissolution time is less than 10 s, and the dissolving-out amount is greater than 90% within 1 min.
2. The saponin extracted from the saponin-containing crude drug by using the extraction method of the triterpenoid saponin of the present invention may keep good pharmacological properties; after being added to a freeze-dried formulation, saponin may still keep good pharmacological properties, such as, blood sugar reduction and lipid lowering, thereby solving the problem of depending on artificial synthesis.
3. The present invention achieves high drug loading capacity of saponin components in a form of a freeze-dried formulation, being up to 950 mg/grain (specification: 1 ml/grain).

### BRIEF DESCRIPTION OF THE DRAWINGS

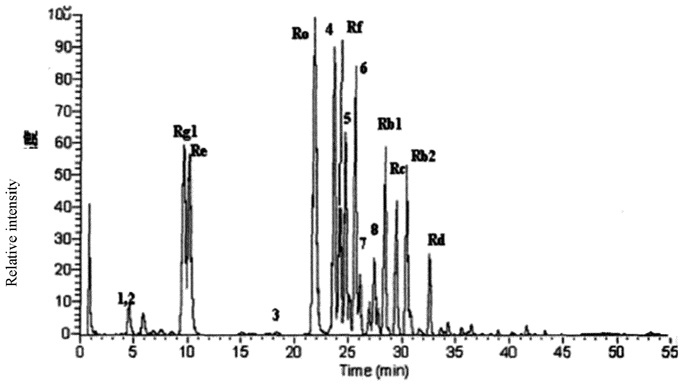

FIG. 1 shows a chromatogram of total ion current of malonyl ginsenoside HPLC-MS of an extract;

In the drawing: 1-malonyl ginsenoside Rg1, 2-malonyl ginsenoside Re, 3-malonyl ginsenoside Rf, 4-malonyl ginsenoside Rb1, 5-malonyl ginsenoside Rc, 6-malonyl ginsenoside Rb2, 7-malonyl ginsenoside Rb3, and 8-malonyl ginsenoside Rd.

### DETAILED DESCRIPTION OF THE INVENTION

The present invention will be further described in details with reference to the detailed examples. It should be understood that the detailed examples described herein are merely used for explaining the present invention, but not intended for limiting thereto.

### Example groups

### Group A experiment:

A method for extracting a triterpenoid saponin includes the following steps:
1) root of ginseng or American ginseng, or "Rhizome" position (the portion of subterraneous stem) of ginseng or American ginseng was taken, cleaned and cut up, then 10 times pure water was added;
2) fragments of the saponin-containing crude drug were homogenized and squeezed at a low-temperature environment of 0°C to obtain a decoction A and a dreg B;
3) the dreg B was taken and extracted for 30min at -30°C by a CO₂ supercritical extraction process to obtain an extract C, and residues were discarded, where an entrainer was ethyl alcohol;
4) the decoction A was taken and subjected to passing through a macroporous resin column, washed with water to remove impurities, and triterpenoid saponin was eluted with 30% ethyl alcohol, sampling was performed and a target product was determined by thin-layer chromatography, a target segment was collected and concentrated under reduced pressure at 0°C until there was no alcoholic taste, and freeze-dried to obtain malonyl ginsenosides Rg1, Re, Rf, Rb1, Rc, Rb2, Rb3 and Rd;
5) the extract C was taken and subjected to passing through a macroporous resin column, washed with water to remove impurities, and the triterpenoid saponin was eluted with 30% ethyl alcohol, sampling was performed and a target product was determined by thin-layer chromatography, a target segment was collected, and concentrated under reduced pressure at 0°C until there was no alcoholic taste, and freeze-dried to obtain malonyl ginsenosides Rg1, Re, Rf, Rb1, Rc, Rb2, Rb3 and Rd.

The malonyl ginsenosides obtained in the steps 4) and 5) were mixed to obtain a chromatogram of total ion current of malonyl ginsenoside HPLC-MS as shown in FIG. 1.

Malonyl ginsenosides Rg1, Re, Rf , Rb1, Rc, Rb2, Rb3 and Rd may be also obtained by extracting the root, stem leaf, flower or any mixture of Panax notoginseng using the same technology. In the group A experiment, the malonyl ginsenosides (including Rg1, Re, Rf, Rb1, Rc, Rb2, Rb3 and Rd) at the root of ginseng or American ginseng, or "Rhizome" position of ginseng or American ginseng had an extraction ratio of 80-83%.

### Group B experiment:

A method for extracting the triterpenoid saponin includes the following steps:
1) the root, stem leaf, flower or any mixture of Gynostemma pentaphyllum was taken, cleaned and cut up, then 5 times pure water was added; 1
2) fragments of the saponin-containing crude drug were homogenized and squeezed at a low-temperature environment of 50°C to obtain a decoction A and a dreg B;
3) the dreg B was taken and extracted for 100 min at -40°C by a CO₂ supercritical extraction process to obtain an extract C, and residues were discarded, where an entrainer was ethyl alcohol;
4) the decoction A was taken and subjected to passing through a macroporous resin column, washed with water to remove impurities, and the triterpenoid saponin was eluted with 100% ethyl alcohol, sampling was performed and a target product was determined by thin-layer chromatography, a target segment was collected and concentrated under reduced pressure at 55°C until there was no alcoholic taste, and freeze-dried to obtain Gynostemma pentaphyllum saponin;
5) the extract C was taken and subjected to passing through a macroporous resin column, washed with water to remove impurities, and the triterpenoid saponin was eluted with 100% ethyl alcohol, sampling was performed and a target product was determined by thin-layer chromatography, a target segment was collected, and concentrated under reduced pressure at 55°C until there was no alcoholic taste, and freeze-dried to obtain Gynostemma pentaphyllum saponin.

In the group B experiment, the Gynostemma pentaphyllum saponin in the root, stem leaf, flower or any mixture of Gynostemma pentaphyllum had an extraction ratio of 81-85%.

Gynostemma pentaphyllum saponin obtained in the group B experiment was verified by using HPLC-MS to obtain a chromatogram of total ion current which was consistent with the standard chromatogram reported by relevant literature.

### Group C experiment:

A method for extracting a triterpenoid saponin includes the following steps:
1) the root, stem leaf, flower or any mixture of Astragalus membranaceus was taken, cleaned and cut up, then 5 times pure water was added;
2) fragments of the saponin-containing crude drug were homogenized and squeezed at a low-temperature environment of 50°C to obtain a decoction A and a dreg B;
3) the dreg B was taken and extracted for 60min at 20°C by a CO₂ supercritical extraction process to obtain an extract C, and residues were discarded, where an entrainer was ethyl alcohol;
4) the decoction A was taken and subjected to passing through a macroporous resin column, washed with water to remove impurities, and the triterpenoid saponin was eluted with 60% ethyl alcohol, sampling was performed and a target product was determined by thin-layer chromatography, a target segment was collected and concentrated under reduced pressure at 25°C until there was no alcoholic taste, and freeze-dried to obtain Astragalus lanuginosus saponins (X, XIV, XV, and XVI);
5) the extract C was taken and subjected to passing through a macroporous resin column, washed with water to remove impurities, and the triterpenoid saponin was eluted with 60% ethyl alcohol, sampling was performed and a target product was determined by thin-layer chromatography, a target segment was collected, and concentrated under reduced pressure at 25°C until there was no alcoholic taste, and freeze-dried to obtain Astragalus lanuginosus saponins (X, XIV, XV, and XVI);

In the group C experiment, the Astragalus lanuginosus saponins (X, XIV, XV, and XVI) in the root, stem leaf, flower or any mixture of Astragalus membranaceus had an extraction ratio of 85-90%.

Astragalus lanuginosus saponins (X, XIV, XV, and XVI) obtained in the group C experiment was verified by using HPLC-MS to obtain a chromatogram of total ion current which was consistent with the standard chromatogram reported by relevant literature.

### Group D experiment:

A method for extracting a triterpenoid saponin includes the following steps:
1) the root, stem leaf, flower or any mixture of Codonopsis pilosula was taken, cleaned and cut up, then 1-fold pure water was added;
2) fragments of the saponin-containing crude drug were homogenized and squeezed at a low-temperature environment of 20°C to obtain a decoction A and a dreg B;
3) the dreg B was taken and extracted for 50 min at 30°C by a CO₂ supercritical extraction process to obtain an extract C, and residues were discarded, where an entrainer was ethyl alcohol;
4) the decoction A was taken and subjected to passing through a macroporous resin column, washed with water to remove impurities, and the triterpenoid saponin was eluted with 40% ethyl alcohol, sampling was performed and a target product was determined by thin-layer chromatography, a target segment was collected and concentrated under reduced pressure at 40°C until there was no alcoholic taste, and freeze-dried to obtain Codonopsis pilosula saponin A-G, and Aster tataricus saponinHb, and Condonpsis lanceolata saponin I-III;
5) the extract C was taken and subjected to passing through a macroporous resin column, washed with water to remove impurities, and the triterpenoid saponin was eluted with 40% ethyl alcohol, sampling was performed and a target product was determined by thin-layer chromatography, a target segment was collected, and concentrated under reduced pressure at 40°C until there was no alcoholic taste, and freeze-dried to obtain Codonopsis pilosula saponin A-G, and Aster tataricus saponin Hb, and Condonpsis lanceolata saponin I-III.

In the group D experiment, the Codonopsis pilosula saponin A-G, and Aster tataricus saponin Hb, and Condonpsis lanceolata saponin I-III in the root, stem leaf, flower or any mixture of Codonopsis pilosula had an extraction ratio of 82-86%.

Codonopsis pilosula saponin A-G, and Aster tataricus saponin Hb, and Condonpsis lanceolata saponin I-III obtained in the group C experiment were verified by using HPLC-MS to obtain a chromatogram of total ion current which was consistent with the standard chromatogram reported by relevant literature.

### Example 1

A freeze-dried formulation was provided, and the freeze-dried formulation served as an oral formulation for use; the triterpenoid saponin in the freeze-dried formulation was malonyl ginsenoside Re having a percentage content of 10%, and the binding agent was Aureobasidium pullulans polysaccharide having a percentage content of 3%.

The freeze-dried formulation was prepared by the following steps:
a) water, malonyl ginsenoside Re and Aureobasidium pullulans polysaccharide were mixed to form a primary prepared solution, then the volume of the solution was fixed and the solution was degassed;
b) the primary prepared solution obtained in the step a) was pumped into a 1 ml sheet-like forming die by using a quantitative filling pump for degassing;
c) a side product obtained in the step b) was freeze-dried, and the solvent was removed to obtain an oral freeze-dried formulation.

The oral formulation had hypoglycemic functions and had a disintegration time within 3 s; the dissolving-out amount may be up to 95% within a dissolution rate of 30 s. In this example, when malonyl ginsenoside Re was replaced with other types of malonyl ginsenosides, the obtained oral formulation had the same properties; the disintegration time was within 3 s, and the dissolving-out amount may be up to 95% within a dissolution rate of 30 s.

In this example, when the formula was modified as follows: the content of malonyl ginsenoside Re was 0.004%, and the content of Aureobasidium pullulans polysaccharide was 0.01%, or the content of malonyl ginsenoside Re was 50%, and the content of Aureobasidium pullulans polysaccharide was 50%, the oral formulation having the same effect may be still obtained.

### Example 2

A freeze-dried formulation was provided, and the freeze-dried formulation served as an oral formulation for use; the triterpenoid saponin in the freeze-dried formulation was 10% malonyl ginsenoside Rg1 and 20% malonyl ginsenoside Rb1, 30% in total; the binding agent was guar gum (percentage content was 0.5%) and butanediol (percentage content was 15%); the adjuvant was a framework propping agent (modified cassava starch) with a percentage content of 5%. The oral formulation had a disintegration time within 3 s; the dissolving-out amount may be up to 95% within a dissolution rate of 30 s.

The freeze-dried formulation was prepared by the following steps:
a) preparation of a soft ice mixture: malonyl ginsenoside Rg1, malonyl ginsenoside Rb1, guar gum and butanediol were added water and mixed according to a preset ratio to obtain a primary prepared solution, then the primary prepared solution was frozen to obtain a soft ice mixture 1;
b) the soft ice mixture 1 was shaped by a spherical die (specification: 0.6 ml) to obtain a shaped mixture 2, and demolding was performed;
g) the mixture 2 was freeze-dried to obtain a freeze-dried formulation;
   the oral formulation had hypoglycemic functions and had a disintegration time within 3 s; the dissolving-out amount may be up to 95% within a dissolution rate of 30 s.

In this example, when the formula was modified as follows: the triterpenoid saponin was 0.002% malonyl ginsenoside Rg1 and 0.002% malonyl ginsenoside Rb1, and guar gum (percentage content was 0.005%) and butanediol (percentage content was 0.005%); or triterpenoid saponin was 20% malonyl ginsenoside Rg1 and 10% malonyl ginsenoside Rb1, and guar gum (percentage content was 20%) and butanediol (percentage content was 15%), the oral formulation having the same properties may be still obtained.

### Example 3

A freeze-dried formulation was provided, and the freeze-dried formulation served as an oral formulation for use; the triterpenoid saponin in the freeze-dried formulation was 30% platycodin E, and the binding agent was modified starch (percentage content was 30%); the adjuvant was an antioxidant (vitamin C having a percentage content of 7%), and the active ingredient was ginseng polysaccharide (percentage content was 5%). The oral formulation had a disintegration time within 3 s; the dissolving-out amount may be up to 90% within a dissolution rate of 50 s.

The freeze-dried formulation was prepared by the following steps:
a) preparation of a soft ice mixture: platycodin E, modified starch, vitamin C and water were mixed according to a preset ratio to obtain a primary prepared solution, then the primary prepared solution was frozen to obtain a soft ice mixture;
b) ginseng polysaccharide was used as a dry powder;
c) the soft ice mixture, and the ice powder were mixed to obtain a mixture of all soft ice;
d) the soft ice mixture was shaped with an ellipsoidal die to obtain a shaped mixture, and demolding was performed;
e) the mixture was freeze-dried to obtain an oral formulation.

The oral formulation had cardiovascular nursing functions and a disintegration time within 3 s; the dissolving-out amount may be up to 93% within a dissolution rate of 30 s.

In this example, when the formula was modified as follows: the triterpenoid saponin was 10% platycodin E and modified starch (percentage content was 1%); the adjuvant was an antioxidant (vitamin C having a percentage content of 0.01%), the active ingredient was ginseng polysaccharide (percentage content was 50%), or the formula was modified as follows: triterpenoid saponin was 20% platycodin E and modified starch (percentage content was 10%); the adjuvant was an antioxidant (vitamin C having a percentage content of 50%), the active ingredient was ginseng polysaccharide (percentage content was 0.01%), the oral formulation having the same properties may be still obtained.

Based on Examples 1-3, the type and content of the triterpenoid saponin, the binding agent, the adjuvant or the active ingredients are changed within the scope of the present invention, which may achieve that the oral formulation has a disintegration time less than 3 s and a dissolving-out amount within 1 min greater than 90%.

### Example 4

A freeze-dried formulation was provided, and the freeze-dried formulation served as a solid beverage (granules or fast dissolving tablets were available) for use, and had lipid-lowering functions; the triterpenoid saponin in the freeze-dried formulation was Codonopsis pilosula saponin A (percentage content was 70%), the binding agent was sodium hyaluronate (percentage content was 10%). The solid beverage had a disintegration time of 8 s, a dissolution time less than 12 s, and a dissolving-out amount greater than 90% within 30 s.

The freeze-dried formulation was prepared by the following steps:
a) water, Codonopsis pilosula saponin A or polyamino acid were mixed according to a preset ratio to form a primary prepared solution, then volume of the solution was fixed and the solution was degassed;
b) the primary prepared solution obtained in the step a) was pumped into a 1 ml sheet-like forming die by using a quantitative filling pump for degassing;
c) a side product obtained in the step b) was freeze-dried, and the solvent was removed to obtain a solid beverage.

Based on the example, when the formula was modified as follows: the triterpenoid saponin was Codonopsis pilosula saponin A (percentage content was 50%), the binding agent was sodium hyaluronate (percentage content was 20%), or the formula was modified as follows: the triterpenoid saponin was Codonopsis pilosula saponin A (percentage content was 95%), the binding agent was sodium hyaluronate (percentage content was 0.01%), the solid beverage having the same properties may be still obtained.

### Example 5

A freeze-dried formulation was provided, and the freeze-dried formulation served as a solid beverage (granules or fast dissolving tablets were available) for use, and had lipid-lowering functions; the triterpenoid saponin in the freeze-dried formulation was Gynostemma pentaphyllum saponin (percentage content was 95%), the binding agent was polyamino acid (percentage content was 1%); the adjuvant was a pH regulator (citric acid having a percentage content of 1%), and an essence (a coffee flavor having a percentage content of 1%). The solid beverage had a disintegration time of 6 s, a dissolving time less than 10 s, and the dissolving-out amount was greater than 95% within 30 min.

The freeze-dried formulation was prepared by the following steps:
a) preparation of a soft ice mixture: Gynostemma pentaphyllum saponin, polyamino acid, pH regulator and essence were mixed with water according to a preset ratio to obtain a primary prepared solution, then the primary prepared solution was frozen to obtain a soft ice mixture 1;
b) the soft ice mixture 1 was shaped by a cartoon-image die (specification: 1 ml) to obtain a shaped mixture 2, and demolding was performed;
c) the mixture 2 was freeze-dried to obtain a freeze-dried formulation;
   based on the example, when the formula was modified as follows: the triterpenoid saponin was Gynostemma pentaphyllum saponin (percentage content was 85%), the binding agent was polyamino acid (percentage content was 0.01%), or the formula was modified as follows: the triterpenoid saponin was Gynostemma pentaphyllum saponin (percentage content was 50%), the binding agent was polyamino acid (percentage content was 20%), the solid beverage having the same properties may be still obtained.

### Example 6

A freeze-dried formulation was provided, and the freeze-dried formulation served as a solid beverage (granules or fast dissolving tablets were available) for use, and had antifatigue functions; the triterpenoid saponin in the freeze-dried formulation was Aster tataricus saponin Hb (percentage content was 80%), the binding agent was an inorganic matter gel (percentage content was 10%); the adjuvant was sodium carboxymethyl starch (percentage content was 2%), and the active ingredient was arginine (percentage content was 1%) and pilose antler polypeptide (percentage content was 1%). The solid beverage had a disintegration time of 5 s, a dissolution time less than 12 s, and a dissolving-out amount greater than 95% within 50 s.

The freeze-dried formulation was prepared by the following steps:
a) preparation of a soft ice mixture: Aster tataricus saponin Hb, sodium carboxymethyl starch and water were mixed according to a preset ratio to obtain a primary prepared solution, then the primary prepared solution was frozen to obtain a soft ice mixture;
b) arginine, pilose antler polypeptide and effervescing agent served as a dry powder;
c) the soft ice mixture, and the ice powder were mixed to obtain a mixture of all soft ice;
d) the soft ice mixture was shaped with a tablet-shaped die to obtain a shaped mixture, and demolding was performed;
e) the mixture was freeze-dried to obtain a solid beverage.

Based on the example, when the formula was modified as follows: Aster tataricus saponin Hb (percentage content was 70%), the binding agent was an inorganic matter gel (percentage content was 10%), or the formula was modified as follows: Aster tataricus saponin Hb (percentage content was 80%), the binding agent was inorganic matter gel (percentage content was 0.01%), the solid beverage having the same properties may be still obtained.

Based on Examples 4-6, the type and content of the triterpenoid saponin, the binding agent, the adjuvant or the active ingredient were changed within the scope of the present invention, which may achieve that the solid beverage had a disintegration time less than 10 s, a dissolution time less than 15 s, and a dissolving-out amount greater than 90% within 1 min.

### Example 7

A freeze-dried formulation was provided, and the freeze-dried formulation served as a non-washing skin care product (specifically an anti-mature skin whitening essence) for use, and had anti-mature skin and whitening functions; the triterpenoid saponin in the freeze-dried formulation was Condonpsis lanceolata saponin I (percentage content was 0.1%), the binding agent was an cellulose ether (percentage content was 2%); the adjuvant was a skin penetration enhancer (percentage content was 0.2%) and a skin feeling improver (percentage content was 5%), and the active ingredient was steroid saponin (percentage content was 0.1%) and hydrolyzed pearl (percentage content was 1%). The non-washing skin care product had a disintegration time of 5 s, a dissolution time less than 12 s, and a dissolving-out amount greater than 95% within 50 s.

The freeze-dried formulation was prepared by the following steps:
a) preparation of a soft ice mixture: Condonpsis lanceolata saponin I, cellulose ether and water were mixed according to a preset ratio to obtain a primary prepared solution, then the primary prepared solution was frozen to obtain a soft ice mixture;
b) the skin penetration enhancer, the skin feeling improver, steroid saponin and hydrolyzed pearl served as a dry powder;
c) the soft ice mixture, and the ice powder were mixed to obtain a mixture of all soft ice;
d) the soft ice mixture was shaped with a tablet-shaped die to obtain a shaped mixture, and demolding was performed;
e) the mixture was freeze-dried to obtain a non-washing skin care product.

During use procedure, the non-washing skin care product was dissolved by 1-3 ml water, astringent or essence to obtain an essence, a cream or a body lotion applied on the skin surface of the corresponding position of a body, thus achieving the anti-mature skin and whitening efficacy.

Based on the example, when the formula was modified as follows: the triterpenoid saponin was Condonpsis lanceolata saponin I (percentage content was 0.004%), and the binding agent was an cellulose ether (percentage content was 0.01%); or the triterpenoid saponin was Condonpsis lanceolata saponin I (percentage content was 30%), and the binding agent was an cellulose ether (percentage content was 50%); or the triterpenoid saponin was Condonpsis lanceolata saponin I (percentage content was 10%), and the binding agent was an cellulose ether (percentage content was 10%); or the triterpenoid saponin was Condonpsis lanceolata saponin I (percentage content was 0.1%), and the binding agent was an cellulose ether (percentage content was 3%), the non-washing skin care product having the same properties may be still obtained.

Based on Example 7, the type and content of the triterpenoid saponin, the binding agent, the adjuvant or the active ingredient were changed within the scope of the present invention, which may achieve that the freeze-dried formulation had a disintegration time less than 5 s, a dissolution time less than 12 s, and a dissolving-out amount greater than 95% within 50 s.

The package in the above Examples 1-3 may be a double-aluminum, aluminum plastic or high-barrier high molecular blister package; and the package in Examples 4-6 may be a glass or metal container equipped with an aluminum plastic seal or a film seal. The package in the Example 7 may be a pop can made of a metal.

What is stated above are merely preferred embodiments of the present invention; it should be indicated that a person skilled in the art may make several improvements and embellishments within the principle of the present invention; these improvements and embellishments shall be also regarded within the protection scope of the present invention.

## Claims

1. A freeze-dried formulation, wherein a triterpenoid saponin has a mass percentage of 0.004-95% in the freeze-dried formulation, and a binding agent has a mass percentage of 0.01-99% in the freeze-dried formulation;
the freeze-dried formulation is freeze-dried by a primary prepared solution containing the triterpenoid saponin and the binding agent, wherein
when the triterpenoid saponin has a mass percentage of 0.001%-50% in the primary prepared solution, the binding agent has a mass percentage of 0.01%-50% in the primary prepared solution, and a mass of the triterpenoid saponin increases with an increase of a mass of the binding agent;
when the triterpenoid saponin has a mass percentage of 50%-95% in the primary prepared solution, the binding agent has a mass percentage of 0.01%-20% in the primary prepared solution, and the mass of the triterpenoid saponin decreases with the increase of the mass of the binding agent.

2. A freeze-dried formulation, wherein the freeze-dried formulation is freeze-dried by a primary prepared solution containing triterpenoid saponin and a binding agent, and the freeze-dried formulation is an oral formulation, a solid beverage or a non-washing skin care product,
when the freeze-dried formulation is the oral formulation, the triterpenoid saponin has a mass percentage of 0.004% -50% in the primary prepared solution, and the binding agent has a mass percentage of 0.01% -50% in the primary prepared solution ;
when the freeze-dried formulation is the solid beverage, the triterpenoid saponin has a mass percentage of 50% -95% in the primary prepared solution, and the binding agent has a mass percentage of 0.01%-20% in the primary prepared solution.
when the freeze-dried formulation is the non-washing skin care product, the triterpenoid saponin has a mass percentage of 0.004-30% in the primary prepared solution, and the binding agent has a mass percentage of 0.01-50% in the primary prepared solution .

3. The freeze-dried formulation according to claim 2, wherein the oral formulation has a disintegration time less than 3 seconds, and a dissolving-out amount greater than 90% within 1 minute; and the solid beverage has a disintegration time less than 10 seconds, a dissolution time less than 15 seconds, and a dissolving-out amount greater than 90% within 1 minute.

4. The freeze-dried formulation according to claim 1 or 2, wherein the freeze-dried formulation further comprises an adjuvant, and the adjuvant has a mass percentage of 0.01-50% in the primary prepared solution, and the adjuvant is one of a framework propping agent, a disintegrating agent, a skin feeling improver, an antioxidant, a corrigent, an essence, a transmucosal/skin penetration enhancer or a pH regulator or a combination thereof.

5. The freeze-dried formulation according to claim 1 or 2, wherein the freeze-dried formulation further comprises an active ingredient, and the active ingredient has a mass percentage of 0.01-50% in the primary prepared solution, and the active ingredient is one or a combination of steroid saponin, Astragalus extract, ginseng extract, American ginseng extract, Gynostemma pentaphyllum extract, flavone, ginseng polysaccharide, sea cucumber polysaccharide, amino acid, dencichine, pilose antler polypeptide, resveratrol, pearl powder, hydrolyzed pearl, rana japonica oil extract, SOD or hawthorn extract.

6. The freeze-dried formulation according to any one of claim 1 or 2, wherein the binding agent consists of a freeze-dried binding agent and/or a low-temperature binding agent; the freeze-dried binding agent is selected from one or a combination of an artificial or a natural high-molecular polymer, a modified artificial or natural high-molecular polymer, an inorganic matter gel, a polysaccharide, a polysaccharide derivative and salt thereof, sugar alcohol, cellulose ethers, modified starch, albumin or a polyamino acid; the low-temperature binding agent is one of a C1-C16 alcohol, a grease, a surfactant, an artificial or a natural high-molecular polymer, or a modified artificial or natural high-molecular polymer.

7. The freeze-dried formulation according to claim 1 or 2, wherein the triterpenoid saponin is an acidic thermosensitive saponin, preferably, one or a combination of malonyl ginsenoside, Astragalus lanuginosus saponin, platycodin E, Codonopsis pilosula saponin A-G, Aster tataricus saponin Hb, Condonpsis lanceolata saponin I-III or Gynostemma pentaphyllum saponin.

8. The freeze-dried formulation according to claim 1 or 2, wherein the triterpenoid saponin is purified and obtained from a saponin-containing crude drug by a CO₂ supercritical extraction process and a macroporous resin exchange adsorption process, comprising the following steps:
1) taking, cleaning and cutting up the saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 miniutes at -30°C-40°C by the CO₂ supercritical extraction process to obtain an extract C, and discarding residues, wherein an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin1;
5) taking the extract C and passing the extract C through the macroporous resin column, washing with water to remove impurities, and eluting the triterpenoid saponin with 30%-100% ethyl alcohol, sampling and determining the target product by thin-layer chromatography, collecting the target segment, performing concentration under reduced pressure at 0°C-55°C until there is no alcoholic taste, and freeze-drying to obtain a triterpenoid saponin 2;
6) separately using the triterpenoid saponin 1 and the triterpenoid saponin 2 or combining the triterpenoid saponins 1 and 2 to obtain a final triterpenoid saponin product.

9. The freeze-dried formulation according to claim 8, wherein the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, Astragalus membranaceus or Phytolacca acinosa; the saponin-containing crude drug is one or a combination of a root, a stem, a leaf or a flower of a plant.

10. The freeze-dried formulation according to claim 1 or 2, wherein the freeze-dried formulation is prepared by the following steps:
a) mixing a solvent, the triterpenoid saponin and the binding agent to form the primary prepared solution in a form of a solution, an emulsion or a suspension; or mixing a solvent, the triterpenoid saponin, the binding agent and an adjuvant to form the primary prepared solution in the form of a solution, a emulsion or a suspension, then fixing the volume and degassing;
b) using a quantitative filling pump to pump the primary prepared solution obtained in the step a) into a quantitative forming die for degassing;
c) freeze-drying a side product obtained in the step b), and removing the solvent to obtain the freeze-dried formulation;
or the freeze-dried formulation is prepared by the following steps:
a) preparation of a soft ice mixture: mixing the triterpenoid saponin, the binding agent and a solvent to obtain the primary prepared solution, or mixing the triterpenoid saponin, the binding agent, the solvent and the adjuvant to form the primary prepared solution, then freezing the primary prepared solution to obtain a soft ice mixture 1;
b) mixing an active ingredient, the binding agent, the binding agent and the solvent to obtain the primary prepared solution; or mixing the active ingredient, the binding agent, the adjuvant and the solvent to obtain the primary prepared solution, then performing a low-temperature cryogenic grinding or a low-temperature spraying to obtain an ice powder 2;
c) using the active ingredient and the adjuvant as a dry powder 3;
d) using the active ingredient as a dry powder 4;
e) mixing one or more of the soft ice mixture 1, the ice powder 2, the dry powder 3 or the dry powder 4 to obtain a mixture of all soft ice;
f) performing shaping with a certain die to obtain a shaped mixture 5, and performing demolding;
g) freeze-drying the mixture 5 to obtain the freeze-dried formulation;
or the freeze-dried formulation is prepared by the following steps:
a) mixing the solvent, the triterpenoid saponin and the binding agent to form the primary prepared solution in the form of a solution, an emulsion or a suspension; or mixing the solvent, the triterpenoid saponin, the binding agent and the adjuvant to form the primary prepared solution in the form of a solution, an emulsion or a suspension, then fixing the volume and degassing;
b) using a quantitative filling pump, and performing instillation in a capsule, wherein an internal temperature of the capsule is below an eutectic point of the solution, then rapidly freezing the solution when the solution drops to obtain a frozen solution;
c) freeze-drying the frozen solution to obtain the freeze-dried formulation.

11. The freeze-dried formulation according to claim 1 or 2, wherein the freeze-dried formulation may be in a shape of, but not limited to, a tablet shape, a spherical shape, an ellipsoidal shape, or various characters, animals, plants, food, graphic identifiers or cartoon images.

12. An application of the freeze-dried formulation according to claim 1 or 2 in daily chemicals, drugs, health care products and food.

13. A barrier package, wherein the freeze-dried formulation according to claim 1 or 2 is a content of the barrier package.

14. The barrier package according to claim 13, wherein the barrier package is a double-aluminum, an aluminum plastic or a high-barrier high molecular blister package, a glass or metal container equipped with an aluminum plastic seal or a film seal, or a sealed container made of a metal.

15. An application of triterpenoid saponin in a freeze-dried formulation, wherein the triterpenoid saponin is an acidic thermosensitive saponin, and the acidic thermosensitive saponin is a triterpenoid saponin having a structure with carboxyl, a heating temperature higher than 50°C and a loss rate greater than 30%.

16. The application according to claim 15, wherein the triterpenoid saponin is purified and obtained from a saponin-containing crude drug by a CO₂ supercritical extraction process and a macroporous resin exchange adsorption process, comprising the following steps:
1) taking, cleaning and cutting up the saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 miniutes at -30°C-40°C by the CO₂ supercritical extraction process to obtain an extract C, and discarding residues, wherein an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and performing freeze-drying to obtain a triterpenoid saponin 1;
5) taking the extract C and passing the extract C through the macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and freeze-drying to obtain a triterpenoid saponin 2; and
6) separately using the triterpenoid saponin 1 and the triterpenoid saponin 2 or combining the triterpenoid saponins 1 and 2 to obtain a final triterpenoid saponin product.

17. The application according to claim 16, wherein the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, Astragalus membranaceus or Phytolacca acinosa; the saponin crude drug is one or a combination of a root, a stem, a leaf or flower of a plant.

18. A method for extracting triterpenoid saponin, comprising the following steps:
1) taking, cleaning and cutting up a saponin-containing crude drug, and adding 1-10 times water;
2) homogenizing and squeezing fragments of the saponin-containing crude drug at a low-temperature environment of 0°C-50°C to obtain a decoction A and a dreg B;
3) taking the dreg B and extracting the dreg B for 30-100 minutes at -30°C-40°C by a CO₂ supercritical extraction process to obtain an extract C, and discarding residues, wherein an entrainer is ethyl alcohol;
4) taking the decoction A and passing the decoction A through a macroporous resin column, washing with water to remove impurities, and eluting the triterpenoid saponin with 30%-100% ethyl alcohol, sampling and determining a target product by thin-layer chromatography, collecting a target segment, performing concentration under reduced pressure at 0°C-55°C until there is no alcoholic taste, and freeze-drying to obtain a triterpenoid saponin 1;
5) taking the extract C and passing the extract C through the macroporous resin column, washing with water to remove impurities, and eluting the triterpenoid saponin with 30%-100% ethyl alcohol, sampling and determining a target product by thin-layer chromatography, collecting the target segment, performing concentration under reduced pressure at 0°C-55°C until there is no alcoholic taste, and performing freeze-dryingto obtain a triterpenoid saponin 2; and
6) separately using the triterpenoid saponin 1 and the triterpenoid saponin 2 or combining the triterpenoid saponins 1 and 2 to obtain a final triterpenoid saponin product.

19. The method according to claim 18, wherein the saponin-containing crude drug is one or a combination of one or more plants of American ginseng, ginseng, Panax notoginseng, Gynostemma pentaphyllum, Radix bupleuri, Codonopsis pilosula, Platycodon grandiflorum, Polygala tenuifolia, liquorice, Astragalus membranaceus or Phytolacca acinosa; the saponin crude drug is one or a combination of a root, a stem, a leaf or a flower of a plant.

20. A preparation method of a freeze-dried formulation, wherein the freeze-dried formulation is prepared by the following steps:
a) mixing a solvent, a triterpenoid saponin and a binding agent to form a primary prepared solution in a form of a solution, an emulsion or a suspension; or mixing the solvent, the triterpenoid saponin, the binding agent and the adjuvant to form a primary prepared solution in the form of a solution, an emulsion or a suspension, then fixing the volume and degassing;
b) using a quantitative filling pump to pump the primary prepared solution obtained in the step a) into a quantitative forming die for degassing;
c) freeze-drying a side product obtained in the step b), and removing the solvent to obtain the freeze-dried formulation;
or the freeze-dried formulation is prepared by the following steps:
a) preparation of a soft ice mixture: mixing the triterpenoid saponin, the binding agent and the solvent to obtain a primary prepared solution, or mixing the triterpenoid saponin, the binding agent, the solvent and the adjuvant to form a primary prepared solution, then freezing the primary prepared solution to obtain a soft ice mixture 1;
b) mixing an active ingredient, the binding agent and the solvent to obtain a primary prepared solution; or mixing the active ingredient, the binding agent, the adjuvant and the solvent to obtain a primary prepared solution, then performing low-temperature cryogenic grinding or low-temperature spraying to obtain an ice powder 2;
c) using the active ingredient and the adjuvant as a dry powder 3;
d) using the active ingredient as a dry powder 4;
e) mixing one or more of the soft ice mixture 1, the ice powder 2, the dry powder 3 or the dry powder 4 to obtain a mixture of all soft ice;
f) performing shaping with a certain die to obtain a shaped mixture 5, and performing demolding;
g) freeze-drying the mixture 5 to obtain the freeze-dried formulation;
or the freeze-dried formulation is prepared by the following steps:
a) mixing the solvent, the triterpenoid saponin and the binding agent to form a primary prepared solution in the form of a solution, an emulsion or a suspension; or mixing the solvent, the triterpenoid saponin, the binding agent and the adjuvant to form a primary prepared solution in the form of a solution, an emulsion or a suspension, then fixing a volume and degassing;
b) using a quantitative filling pump, and performing instillation in a capsule, wherein an internal temperature of the capsule is below an eutectic point of the solution, then rapidly freezing the solution when the solution drops to obtain a frozen solution;
c) freeze-drying the frozen solution to obtain the freeze-dried formulation.
